# EUROPEAN PATENT APPLICATION

(11) **EP 4 764 771 A1**
(43) Date of publication of application: **24.06.2026**
(21) Application number: 24874922.8
(22) Date of filing: 30.09.2024
(51) Int. Cl.: G06F 1/16, G06F 11/32, G06F 1/3212, H05B 47/16, H05B 45/20

(54) **RING ELECTRONIC DEVICE AND OPERATION METHOD THEREOF**

(30) Priority: 04.10.2023 KR 20230131681; 05.10.2023 KR 20230132876
(71) Applicant: Samsung Electronics Co., Ltd., Suwon-si, Gyeonggi-do 16677 (KR)
(72) Inventor: LEE, Seungyun, Suwon-si Gyeonggi-do 16677 (KR); KIM, Younghoon, Suwon-si Gyeonggi-do 16677 (KR); KIM, Heejin, Suwon-si Gyeonggi-do 16677 (KR); LEE, Jeonghyun, Suwon-si Gyeonggi-do 16677 (KR); LIM, Hyungjoon, Suwon-si Gyeonggi-do 16677 (KR)
(74) Representative: Appleyard Lees IP LLP
(86) International application number: PCT/KR2024/014874
(87) International publication number: WO 2025/075366

(57) **Abstract**

A method for operating a ring electronic device according to one embodiment of the present invention may comprise an operation of monitoring a wearing state of the ring electronic device. The operation method may comprise an operation of checking a remaining battery level of the ring electronic device when the wearing state is changed. The operation method may comprise an operation of displaying the remaining battery level by adaptively blinking a light-emitting unit included in a PPG sensor of the ring electronic device.

## Description

### TECHNICAL FIELD

Embodiments of the present disclosure relate to a ring electronic device and an operation method thereof.

### BACKGROUND ART

A ring-type wearable electronic device (hereinafter, referred to as a ring electronic device) is an electronic device that is worn on a part (e.g., a finger) of a user's body and performs various functions. The ring electronic device may monitor health condition (e.g., a heart rate, a sleep pattern, an activity level, and/or a stress level) of the user using at least one sensor. The ring electronic device may track activity information (e.g., position, posture, and/or motion) of the user. The ring electronic device may be linked to another electronic device and provide the user with notifications (e.g., calls, text messages, and/or emails) of the other electronic device. The ring electronic device may provide a payment function through a near field communication (NFC) function.

The ring electronic device may be designed to have a waterproof function to be wearable during exercise or daily life. The ring electronic device may have shockresistant durability to track activities of the user. The ring electronic device may incorporate convenient and practical functions to further enhance the user's quality of life and help promote healthy habits of the user.

The above information may be presented as the related art to help with the understanding of the disclosure. No arguments or decisions are raised to whether any of the above description is applicable as the prior art related to the present disclosure.

### DISCLOSURE OF THE INVENTION

### TECHNICAL SOLUTIONS

According to an embodiment, an operation method of a ring electronic device includes monitoring a wearing state of the ring electronic device. The operation method may include, when the wearing state changes, checking the remaining battery level of the ring electronic device. The operation method may include displaying the remaining battery level by adaptively blinking a light emitter included in a photoplethysmography (PPG) sensor of the ring electronic device.

According to an embodiment, a ring electronic device includes a wireless communication circuit configured to transmit and receive a wireless signal. The ring electronic device may include a processor operatively connected to the wireless communication circuit. The ring electronic device may include a memory storing instructions. The instructions, when executed individually or collectively by the processor, may cause the ring electronic device to monitor a wearing state of the ring electronic device. The instructions, when executed individually or collectively by the processor, may cause the ring electronic device to, when the wearing state changes, check the remaining battery level of the ring electronic device. The instructions, when executed individually or collectively by the processor, may cause the ring electronic device to display the remaining battery level by adaptively blinking a light emitter included in a PPG sensor of the ring electronic device.

According to an embodiment, an operation method of a ring electronic device includes monitoring shaking information of the ring electronic device. The operation method may include, when shaking of the ring electronic device is detected, checking the remaining battery level of the ring electronic device. The operation method may include displaying the remaining battery level by adaptively blinking a light emitter included in a PPG sensor of the ring electronic device.

According to an embodiment, a ring electronic device includes a wireless communication circuit configured to transmit and receive a wireless signal. The ring electronic device may include a processor operatively connected to the wireless communication circuit. The ring electronic device may include a memory storing instructions. The instructions, when executed individually or collectively by the processor, may cause the ring electronic device to monitor shaking information of the ring electronic device. The instructions, when executed individually or collectively by the processor, may cause the ring electronic device to, when shaking of the ring electronic device is detected, check the remaining battery level of the ring electronic device. The instructions, when executed individually or collectively by the processor, may cause the ring electronic device to display the remaining battery level by adaptively blinking a light emitter included in a PPG sensor of the ring electronic device.

### BRIEF DESCRIPTION OF DRAWINGS

FIG. 1 is a block diagram of an electronic device in a network environment according to an embodiment.
FIG. 2 is a diagram illustrating a structure of a ring electronic device according to an embodiment.
FIG. 3 is a diagram illustrating a photoplethysmography (PPG) sensor according to an embodiment.
FIGS. 4 and 5 are diagrams illustrating an operation method of a ring electronic device according to an embodiment.
FIG. 6 is a diagram illustrating an operation method of a ring electronic device according to an embodiment.

### BEST MODE FOR CARRYING OUT THE INVENTION

Hereinafter, the examples will be described in detail with reference to the accompanying drawings. When describing the embodiments with reference to the accompanying drawings, like reference numerals refer to like elements and a repeated description related thereto will be omitted.

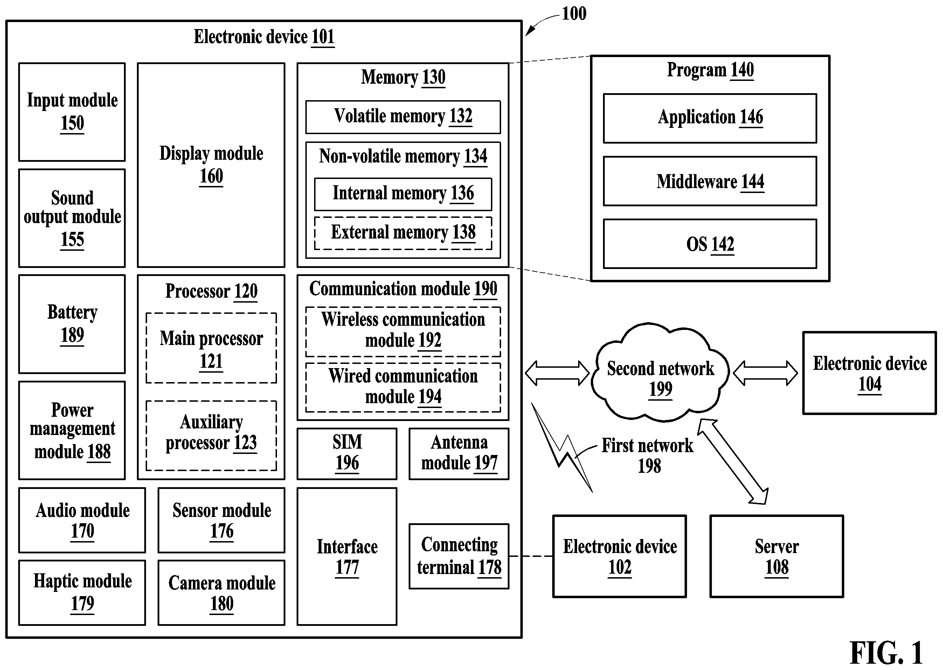

FIG. 1 is a block diagram of an electronic device in a network environment according to an embodiment.

FIG. 1 is a block diagram of an electronic device 101 in a network environment 100 according to an embodiment. Referring to FIG. 1, the electronic device 101 in the network environment 100 may communicate with an electronic device 102 via a first network 198 (e.g., a short-range wireless communication network), or communicate with at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, and a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added to the electronic device 101. In some embodiments, some of the components (e.g., the sensor module 176, the camera module 180, or the antenna module 197) may be integrated as a single component (e.g., the display module 160).

The processor 120 may execute, for example, software (e.g., a program 140) to control at least one other component (e.g., a hardware or software component) of the electronic device 101 connected to the processor 120 and may perform various data processing or computation. According to an embodiment, as at least a part of data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)) or an auxiliary processor 123 (e.g., a graphics processing unit (GPU), a neural processing unit (NPU), an image signal processor (ISP), a sensor hub processor, or a communication processor (CP)) that is operable independently of, or in conjunction with the main processor 121. For example, when the electronic device 101 includes the main processor 121 and the auxiliary processor 123, the auxiliary processor 123 may be adapted to consume less power than the main processor 121 or to be dedicated for a designated function. The auxiliary processor 123 may be implemented separately from the main processor 121 or as a part of the main processor 121.

The auxiliary processor 123 may control at least some of functions or states related to at least one (e.g., the display module 160, the sensor module 176, or the communication module 190) of the components of the electronic device 101, instead of the main processor 121 while the main processor 121 is in an inactive (e.g., sleep) state, or together with the main processor 121 while the main processor 121 is an active state (e.g., executing an application). According to an embodiment, the auxiliary processor 123 (e.g., an ISP or a CP) may be implemented as a portion of another component (e.g., the camera module 180 or the communication module 190) that is functionally related to the auxiliary processor 123. According to an embodiment, the auxiliary processor 123 (e.g., an NPU) may include a hardware structure specified for artificial intelligence (AI) model processing. The AI model may be generated by machine learning. Such learning may be performed by, for example, the electronic device 101 in which an AI model is executed, or performed via a separate server (e.g., the server 108). Learning algorithms may include, but are not limited to, for example, supervised learning, unsupervised learning, semi-supervised learning, or reinforcement learning. The AI model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may additionally or alternatively include a software structure other than the hardware structure.

The memory 130 may store various pieces of data used by at least one component (e.g., the processor 120 or the sensor module 176) of the electronic device 101. The various pieces of data may include, for example, software (e.g., the program 140) and input data or output data for a command related thereto. The memory 130 may include the volatile memory 132 or the non-volatile memory 134.

The program 140 may be stored as software in the memory 130, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for general purposes, such as playing multimedia or playing a recording. The receiver may be used to receive an incoming call. According to an embodiment, the receiver may be implemented separately from the speaker or as a part of the speaker.

The display module 160 may visually provide information to the outside (e.g., a user) of the electronic device 101. The display module 160 may include, for example, a display, a hologram device, or a projector and control circuitry to control a corresponding one of the display, the hologram device, and the projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via an external electronic device (e.g., an electronic device 102) (e.g., a speaker or headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and generate an electric signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used by the electronic device 101 to couple with the external electronic device (e.g., the electronic device 102) directly (e.g., by wire) or wirelessly. According to an embodiment, the interface 177 may include, for example, a high-definition multimedia interface (HDMI), a universal serial bus (USB) interface, a secure digital (SD) card interface, or an audio interface.

The connecting terminal 178 may include a connector via which the electronic device 101 may physically connect to an external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or an electrical stimulus which may be recognized by a user via his or her tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, ISPs, or flashes.

The power management module 188 may manage power supplied to the electronic device 101. According to an embodiment, the power management module 188 may be implemented as, for example, at least a part of a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more CPs that are operable independently of the processor 120 (e.g., an AP) and that support direct (e.g., wired) communication or wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module, or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network #99 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multiple components (e.g., multiple chips) separate from each other. The wireless communication module 192 may identify and authenticate the electronic device 101 in a communication network, such as the first network 198 or the second network 199, using subscriber information (e.g., international mobile subscriber identity (IMSI)) stored in the SIM 196.

The wireless communication module 192 may support a 5G network after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., a mmWave band) to achieve, e.g., a high data transmission rate. The wireless communication module 192 may support various technologies for securing performance on a high-frequency band, such as, e.g., beamforming, massive multiple-input and multiple-output (MIMO), full dimensional MIMO (FD-MIMO), an array antenna, analog beamforming, or a large-scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., an external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in a communication network, such as the first network 198 or the second network 199, may be selected by, for example, the communication module 190 from the plurality of antennas. The signal or the power may be transmitted or received between the communication module 190 and the external electronic device via the at least one selected antenna. According to an embodiment, another component (e.g., a radio frequency integrated circuit (RFIC)) other than the radiating element may be additionally formed as part of the antenna module 197.

According to an embodiment, the antenna module 197 may form a mmWave antenna module. According to an embodiment, the mmWave antenna module may include a PCB, an RFIC disposed on a first surface (e.g., the bottom surface) of the PCB or adjacent to the first surface and capable of supporting a designated high-frequency band (e.g., the mmWave band), and a plurality of antennas (e.g., array antennas) disposed on a second surface (e.g., the top or a side surface) of the PCB, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 and 104 may be a device of a same type as, or a different type from, the electronic device 101. According to an embodiment, all or some of operations to be executed by the electronic device 101 may be executed at one or more external electronic devices (e.g., the external devices 102 and 104, and the server 108). For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request and may transfer an outcome of the performing to the electronic device 101. The electronic device 101 may provide the outcome, with or without further processing of the outcome, as at least part of a reply to the request. To that end, cloud computing, distributed computing, mobile edge computing (MEC), or client-server computing technology may be used, for example. The electronic device 101 may provide ultra-low-latency services using, e.g., distributed computing or MEC. In an embodiment, the external electronic device (e.g., the electronic device 104) may include an Internet-of-Things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

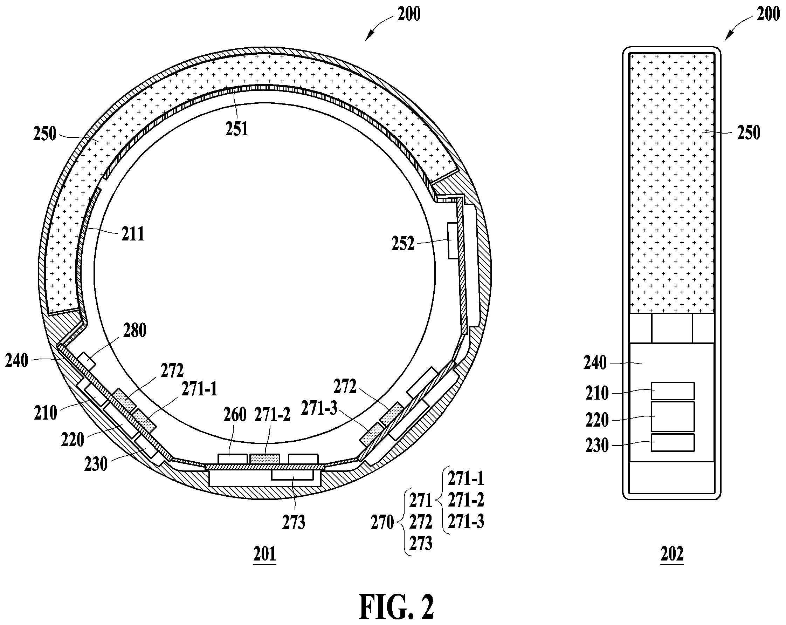

FIG. 2 is a diagram illustrating a structure of a ring electronic device according to an embodiment.

Referring to FIG. 2, a cross-sectional view 201 and a side perspective view 202 of a ring electronic device 200 (e.g., the electronic device 102 of FIG. 1) are illustrated. For example, the ring electronic device 200 may be worn on a finger of a user. The ring electronic device 200 may be a smart ring. The ring electronic device 200 may be designed to have a shape including a hole at a center thereof. The ring electronic device 200 may be formed to include an outer portion and an inner portion. The exterior of the ring electronic device 200 may be implemented to withstand external impacts and scratches. The exterior of the ring electronic device 200 may be implemented using material (e.g., titanium, stainless steel, and/or ceramic) capable of implementing design elements. The interior of the ring electronic device 200 may be a portion in contact with a finger. The interior of the ring electronic device 200 may be implemented using the same material as the exterior, or using a material (e.g., a molding material, transparent plastic, glass, and/or metal) for sensing.

According to an embodiment, the ring electronic device 200 may include a communication circuit 210, an antenna 211, a processor 220, a memory 230, a flexible PCB (FPCB) substrate 240, a battery 250, a charging interface 251, a PMIC 252, an inertial sensor 260, a photoplethysmography (PPG) sensor 270, and a temperature sensor 280.

According to an embodiment, the communication circuit 210 may support establishment of a direct (e.g., wired) communication channel or a wireless communication channel between the ring electronic device 200 and an external electronic device (e.g., the electronic device 101 of FIG. 1). The communication circuit 210 may support communication based on the established communication channel. The communication circuit 210 may support various communication methods (e.g., Bluetooth, Bluetooth low energy (BLE), ZigBee, ANT+, Wi-Fi, cellular communication (LTE, 5G, 6G, or NB-IoT), near field communication (NFC), radiofrequency identification (RFID), ultra-wide band (UWB), and/or GNSS). The communication circuit 210 may operate independently of the processor 220 or may be implemented in an integrated form with the processor 220. The antenna 211 may be an antenna for wireless communication. The antenna 211 may be implemented as a single antenna or as a plurality of antennas. The antenna 211 may also be implemented as a portion of the exterior of the ring electronic device 200.

According to an embodiment, the processor 220 may perform various data processing or operations. According to an embodiment, the processor 220 may store commands or data received from another component (e.g., a sensor (e.g., 260, 270, and/or 280) or the communication circuit 210) in the memory 230, process the commands or data stored in the memory 230, and store result data in the memory 230. According to an embodiment, the processor 220 may be implemented as circuitry (e.g., processing circuitry), such as a system-on-chip (SoC) or an integrated circuit (IC). The processor 220 may include one or more processors. For example, the processor 220 may include a combination of one or more processors, such as a CPU, a GPU, a microprocessor unit (MPU), an AP, and a CP.

According to an embodiment, the processor 220 may include a main processor (e.g., a CPU or an AP) or an auxiliary processor (e.g., a GPU, an NPU, an ISP, a sensor hub processor, or a CP) that is operable independently of or in conjunction with the main processor. The processor 220 may be implemented in an integrated form with another component (e.g., a sensor (e.g., 260, 270, and/or 280) and/or the communication circuit 210).

According to an embodiment, the memory 230 may store various data used by at least one component (e.g., the processor 220 and/or a sensor (e.g., 260, 270, and/or 280)) of the ring electronic device 200. For example, the data may include software (e.g., a program). The data may include input data or output data for commands associated with the software.

According to an embodiment, the memory 230 may include one or more memories. The instructions stored in the memory 230 may be stored in one memory. The instructions stored in the memory 230 may be distributed and stored in a plurality of memories. The instructions stored in the memory 230 may be executed individually or collectively by the processor 220 to cause the ring electronic device 200 to perform and/or control operations of the ring electronic device 200 described with reference to FIGS. 2 to 6. The instructions stored in the memory 230 may be executed individually or collectively by a plurality of processors to cause the ring electronic device 200 to perform and/or control operations of the ring electronic device 200 described with reference to FIGS. 2 to 6.

According to an embodiment, the FPCB substrate 240 may be a flexible electrical circuit substrate. Components (e.g., the processor 220, the memory 230, a sensor (e.g., 260, 270, and/or 280), and/or the battery 250) may be disposed on the FPCB substrate 240. The components disposed on the FPCB substrate 240 may be electrically connected.

According to an embodiment, the battery 250 may store energy for supplying power to the ring electronic device 200. The battery 250 may be charged and discharged and may be formed of various materials (e.g., lithium-ion, mercury, or a dry cell). The battery 250 may include a flexible battery pack to be disposed within the ring electronic device 200. The charging interface 251 may support various charging methods (e.g., a wired charging method and/or a wireless charging method) of the ring electronic device 200. The PMIC 252 may manage power for the ring electronic device 200. The PMIC 252 may appropriately distribute power to the components (e.g., the processor 220 or a sensor (e.g., 260, 270, and/or 280)).

According to an embodiment, the inertial sensor 260 may be a sensor for detecting inertia of the ring electronic device 200. The inertial sensor 260 may include an acceleration sensor and/or a gyroscope sensor. The inertial sensor 260 may include a three-axis acceleration sensor and may be implemented as a three-axis sensor. The inertial sensor 260 may include a three-axis acceleration sensor and a three-axis gyroscope sensor and may be implemented as a six-axis sensor. The inertial sensor 260 may detect motion, gesture, impact, posture, and/or activity of the ring electronic device 200 (or a wearer of the ring electronic device 200). The inertial sensor 260 may monitor shaking information of the ring electronic device 200. The ring electronic device 200 may include a position measurement circuit (e.g., a global positioning system (GPS) circuit) (or a GPS sensor). Through the position measurement circuit (e.g., a GPS circuit) (or a GPS sensor), the ring electronic device 200 may obtain position information thereof.

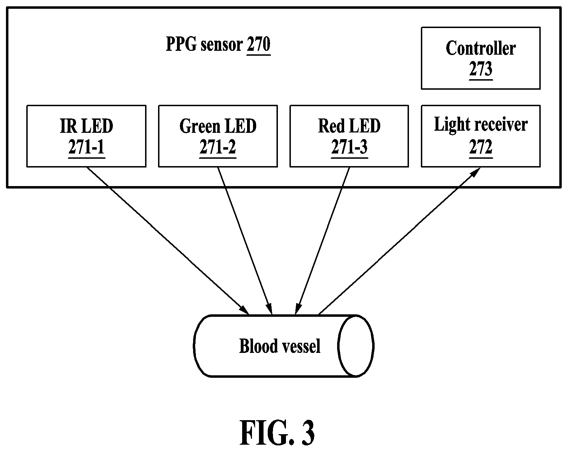

According to an embodiment, the PPG sensor 270 may emit light toward a living body (e.g., a body of a wearer). The PPG sensor 270 may receive absorbed, scattered, and/or reflected light. The PPG sensor 270 may include a light emitter 271, a light receiver 272, and a controller 273. The PPG sensor 270 will be described in detail with reference to FIG. 3.

According to an embodiment, the temperature sensor 280 may be a sensor configured to measure the temperature of a living body or a component. The temperature sensor 280 may measure temperature in a contact or non-contact manner. The temperature sensor 280 may store a measured temperature value in the memory 230. The temperature sensor 280 may transmit the measured temperature value to the processor 220 such that the processor 220 estimates a skin temperature. The ring electronic device 200 may include a sensor (e.g., a wearing detection sensor) configured to detect a finger of a user.

According to an embodiment, a sensor (e.g., 260, 270, 280, and/or the wearing detection sensor) may be selectively activated according to an operation mode of the ring electronic device 200. For example, in a mode (e.g., a health tracking mode) in which the ring electronic device 200 is worn on a finger of a user and tracks health condition and/or activity information of the user (e.g., a wearer of the ring electronic device 200), all sensors (e.g., 260, 270, 280, and the wearing detection sensor) may be activated. For example, when the user's finger is not detected, at least a portion of health sensors (e.g., 260, 270, and 280) may be deactivated, and only the wearing detection sensor may be activated.

FIG. 3 is a diagram illustrating a PPG sensor according to an embodiment.

Referring to FIG. 3, the PPG sensor 270 may include light emitters 271-1, 271-2, and 272-3, the light receiver 272, and the controller 273. The PPG sensor 270 may emit light toward a living body (e.g., a body of a wearer) (e.g., a blood vessel) through the light emitters 271-1, 271-2, and 271-3. The light emitters 271-1, 271-2, and 271-3 may emit light in various wavelength bands. The light emitter 271-1 may emit infrared light, the light emitter 271-2 may emit green light in a visible wavelength band, and the light emitter 271-3 may emit red light in a visible wavelength band. The light emitters 271-1, 271-2, and 271-3 may be implemented as various elements (e.g., a light-emitting diode (LED), a laser diode, or a vertical-cavity surface-emitting laser (VCSEL)).

According to an embodiment, the PPG sensor 270 may receive absorbed, scattered, and/or reflected light through the light receiver 272. The light receiver 272 may receive light resulting from reflection or transmission of the emitted light. The light receiver 272 may be implemented as a photodiode and/or a complementary metaloxide-semiconductor (CMOS)-based image sensor. The light receiver 272 may convert the received light based on an analog-to-digital converter (ADC). The light receiver 272 may store the result of converting the received light in a memory (e.g., the memory 230 of FIG. 2) or a sensor buffer (not shown).

According to an embodiment, the PPG sensor 270 may control the light emitters 271-**1,** 271-2, and 271-3 and the light receiver 272 through the controller 273. The controller 273 may process (e.g., convert received light and store a converted result in the memory 230) data.

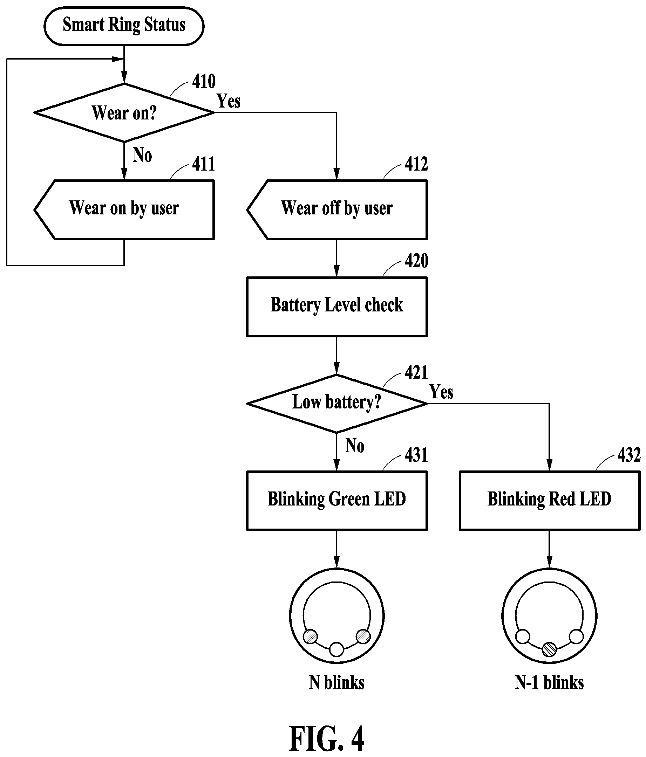

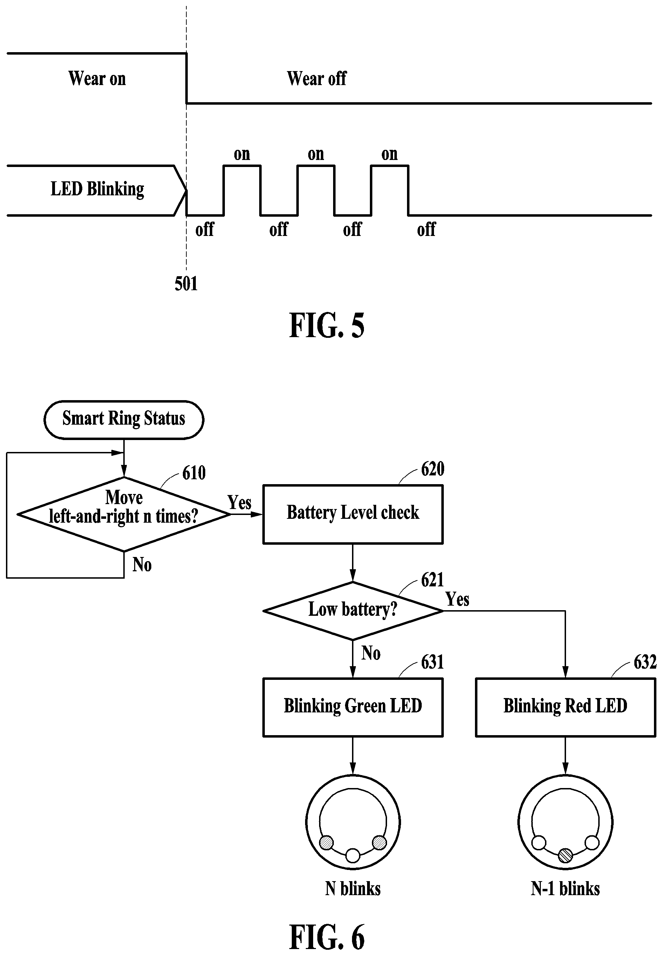

FIGS. 4 and 5 are diagrams illustrating an operation method of a ring electronic device according to an embodiment.

Referring to FIG. 4, operations 410 to 432 may be performed sequentially, but not necessarily. For example, the order of operations 410 to 432 may be changed, or at least two of operations 410 to 432 may be performed in parallel.

In operation 410, the ring electronic device (e.g., the ring electronic device 200 of FIG. 2) according to an embodiment may determine a wearing state of the ring electronic device 200. As described above with reference to FIG. 2, the wearing state of the ring electronic device 200 may be monitored based on a sensor (e.g., a wearing detection sensor) configured to detect a finger of a user.

In operation 411, the ring electronic device 200 may detect that the wearing state of the ring electronic device 200 is maintained (e.g., worn). The ring electronic device 200 may continue monitoring the wearing state. In operation 412, the ring electronic device 200 may detect that the wearing state of the ring electronic device 200 is changed (e.g., not worn). Referring to FIG. 5, when detecting a change in the wearing state (e.g., not worn) (e.g., operation 501), the ring electronic device 200 may adaptively blink a light emitter (e.g., the light emitter 271 of FIG. 2) included in a PPG sensor (e.g., the PPG sensor 270 of FIG. 2).

In operation 420, the ring electronic device 200 may first check a remaining battery level. The remaining battery level may be collected at a predetermined interval (e.g., several milliseconds). The remaining battery level may be expressed as a percentage between 1 and 100.

In operation 421, the ring electronic device 200 may compare a predetermined threshold with the remaining battery level (e.g., N%). The ring electronic device 200 may determine whether the remaining battery level is less than the predetermined threshold.

In operation 431, when the remaining battery level is greater than or equal to the predetermined threshold, the ring electronic device 200 may blink a green LED (e.g., the light emitter 271-2 of FIG. 2) included in the PPG sensor 270. In operation 432, when the remaining battery level is less than the predetermined threshold, the ring electronic device 200 may blink a red LED (e.g., the light emitter 271-3 of FIG. 2) included in the PPG sensor 270.

According to an embodiment, the ring electronic device 200 may blink the light emitter 271 based on the remaining battery level. When the remaining battery level (e.g., N%) is greater than or equal to the predetermined threshold, the ring electronic device 200 may blink the green LED 271-2 the number of times (e.g., N times) corresponding to the remaining battery level. When the remaining battery level (e.g., N%) is less than the predetermined threshold, the ring electronic device 200 may blink the red LED 271-3 the number of times (e.g., N-1 times) equal to the remaining battery level minus 1.

According to an embodiment, the ring electronic device 200 may display the remaining battery level of the ring electronic device 200 without a display member on an outer portion thereof. The ring electronic device 200 may display the remaining battery level based on a PPG sensor on an inner portion (e.g., a portion in contact with a finger) thereof. The ring electronic device 200 may vary the number of blinks of a light emitter (e.g., a light emitter of a PPG sensor) and a color of blinking light based on the remaining battery level. The ring electronic device 200 may intuitively display to a user whether charging is required. Even during exercise, the ring electronic device 200 may allow the user to check a battery state of the ring electronic device 200 without an electronic device (e.g., the electronic device 101 of FIG. 1). The user may conveniently check the battery state of the ring electronic device 200 while wearing the ring electronic device 200.

FIG. 6 is a diagram illustrating an operation method of a ring electronic device according to an embodiment.

Referring to FIG. 6, operations 610 to 632 may be performed sequentially, but not necessarily. For example, the order of operations 610 to 632 may be changed, or at least two of operations 610 to 632 may be performed in parallel.

In operation 610, the ring electronic device (e.g., the ring electronic device 200 of FIG. 2) according to an embodiment may monitor shaking information (e.g., left-and-right shaking) of the ring electronic device 200. The shaking information of the ring electronic device 200 may be monitored based on an inertial sensor (e.g., the inertial sensor 260 of FIG. 2).

In operation 620, the ring electronic device 200 may check a remaining battery level when shaking of the ring electronic device 200 is detected. The remaining battery level may be collected at a predetermined interval (e.g., several milliseconds). The remaining battery level may be expressed as a percentage between 1 and 100.

In operation 621, the ring electronic device 200 may compare a predetermined threshold with the remaining battery level (e.g., N%). The ring electronic device 200 may determine whether the remaining battery level is less than the predetermined threshold.

In operation 631, when the remaining battery level is greater than or equal to the predetermined threshold, the ring electronic device 200 may blink a green LED (e.g., the light emitter 271-2 of FIG. 2) included in a PPG sensor (e.g., the PPG sensor 270 of FIG. 2). In operation 632, when the remaining battery level is less than the predetermined threshold, the ring electronic device 200 may blink a red LED (e.g., the light emitter 271-3 of FIG. 2) included in the PPG sensor 270.

According to an embodiment, the ring electronic device 200 may blink the light emitter 271 based on the remaining battery level. When the remaining battery level (e.g., N%) is greater than or equal to the predetermined threshold, the ring electronic device 200 may blink the green LED 271-2 the number of times (e.g., N times) corresponding to the remaining battery level. When the remaining battery level (e.g., N%) is less than the predetermined threshold, the ring electronic device 200 may blink the red LED 271-3 the number of times (e.g., N-1 times) equal to the remaining battery level minus 1.

According to an embodiment, the ring electronic device 200 may display the remaining battery level when left-and-right shaking is detected after the ring electronic device 200 is removed. Even without an electronic device (e.g., the electronic device 101 of FIG. 1), the ring electronic device 200 may allow a user to check a battery state of the ring electronic device 200.

According to an embodiment, the ring electronic device 200 may display the remaining battery level of the ring electronic device 200 without a display member on an outer portion thereof. The ring electronic device 200 may display the remaining battery level based on a PPG sensor on an inner portion thereof. The ring electronic device 200 may vary the number of blinks of a light emitter (e.g., a light emitter of a PPG sensor) and a color of blinking light based on the remaining battery level. The ring electronic device 200 may intuitively display to a user whether charging is required.

According to an embodiment, an operation method of a ring electronic device (e.g., the electronic device 102 of FIG. 1 or the ring electronic device 200 of FIG. 2) may include monitoring a wearing state of the ring electronic device. The operation method may include, when the wearing state changes, checking the remaining battery level of the ring electronic device. The operation method may include displaying the remaining battery level by adaptively blinking a light emitter (e.g., the light emitter 271 of FIG. 2) included in a PPG sensor (e.g., the PPG sensor 270 of FIG. 2) of the ring electronic device.

According to an embodiment, the displaying of the remaining battery level may include comparing a predetermined threshold with the remaining battery level and adaptively blinking the light emitter included in the PPG sensor.

According to an embodiment, the displaying of the remaining battery level may include, when the remaining battery level is greater than or equal to the predetermined threshold, blinking a green LED (e.g., the light emitter 271-2 of FIG. 2) included in the PPG sensor. The displaying of the remaining battery level may include, when the remaining battery level is less than the predetermined threshold, blinking a red LED (e.g., the light emitter 271-3 of FIG. 2) included in the PPG sensor.

According to an embodiment, the number of blinks of the light emitter is based on the remaining battery level.

According to an embodiment, the blinking of the green LED may include, when the remaining battery level is greater than or equal to the predetermined threshold, blinking the green LED the number of times corresponding to the remaining battery level. The blinking of the red LED may include, when the remaining battery level is less than the predetermined threshold, blinking the red LED the number of times equal to the remaining battery level minus 1.

According to an embodiment, the remaining battery level is collected at a predetermined interval.

According to an embodiment, an operation method of a ring electronic device (e.g., the electronic device 102 of FIG. 1 or the ring electronic device 200 of FIG. 2) may include monitoring shaking information of the ring electronic device. The operation method may include, when shaking of the ring electronic device is detected, checking the remaining battery level of the ring electronic device. The operation method may include displaying the remaining battery level by adaptively blinking a light emitter (e.g., the light emitter 271 of FIG. 2) included in a PPG sensor (e.g., the PPG sensor 270 of FIG. 2) of the ring electronic device.

According to an embodiment, the displaying of the remaining battery level may include comparing a predetermined threshold with the remaining battery level and adaptively blinking the light emitter included in the PPG sensor.

According to an embodiment, the displaying of the remaining battery level may include, when the remaining battery level is greater than or equal to the predetermined threshold, blinking a green LED (e.g., the light emitter 271-2 of FIG. 2) included in the PPG sensor. The displaying of the remaining battery level may include, when the remaining battery level is less than the predetermined threshold, blinking a red LED (e.g., the light emitter 271-3 of FIG. 2) included in the PPG sensor.

According to an embodiment, the number of blinks of the light emitter is based on the remaining battery level.

According to an embodiment, the blinking of the green LED may include, when the remaining battery level is greater than or equal to the predetermined threshold, blinking the green LED the number of times corresponding to the remaining battery level. The blinking of the red LED may include, when the remaining battery level is less than the predetermined threshold, blinking the red LED the number of times equal to the remaining battery level minus 1.

According to an embodiment, the remaining battery level is collected at a predetermined interval.

According to an embodiment, a ring electronic device (e.g., the electronic device 102 of FIG. 1 or the ring electronic device 200 of FIG. 2) may include a wireless communication circuit (e.g., the communication circuit 210 of FIG. 2) configured to transmit and receive a wireless signal. The ring electronic device may include a processor (e.g., the processor 220 of FIG. 2) operatively connected to the wireless communication circuit. The ring electronic device may include a memory (e.g., the memory 230 of FIG. 2) storing instructions. The instructions, when executed individually or collectively by the processor, may cause the ring electronic device to monitor a wearing state of the ring electronic device. The instructions, when executed individually or collectively by the processor, may cause the ring electronic device to, when the wearing state changes, check the remaining battery level of the ring electronic device. The instructions, when executed individually or collectively by the processor, may cause the ring electronic device to display the remaining battery level by adaptively blinking a light emitter (e.g., the light emitter 271 of FIG. 2) included in a PPG sensor (e.g., the PPG sensor 270 of FIG. 2) of the ring electronic device.

According to an embodiment, the instructions, when executed individually or collectively by the processor, cause the ring electronic device to compare a predetermined threshold with the remaining battery level and adaptively blink the light emitter included in the PPG sensor.

According to an embodiment, the instructions, when executed individually or collectively by the processor, cause the ring electronic device to, when the remaining battery level is greater than or equal to the predetermined threshold, blink a green LED (e.g., the light emitter 271-2 of FIG. 2) included in the PPG sensor. The instructions, when executed individually or collectively by the processor, cause the ring electronic device to, when the remaining battery level is less than the predetermined threshold, blink a red LED (e.g., the light emitter 271-3 of FIG. 2) included in the PPG sensor.

According to an embodiment, the number of blinks of the light emitter is based on the remaining battery level.

According to an embodiment, the instructions, when executed individually or collectively by the processor, cause the ring electronic device to, when the remaining battery level is greater than or equal to the predetermined threshold, blink the green LED the number of times corresponding to the remaining battery level. The instructions, when executed individually or collectively by the processor, cause the ring electronic device to, when the remaining battery level is less than the predetermined threshold, blink the red LED the number of times equal to the remaining battery level minus 1.

According to an embodiment, the remaining battery level is collected at a predetermined interval.

The instructions, when executed individually or collectively by the processor, may cause the ring electronic device to monitor a wearing state of the ring electronic device based on shaking information of the ring electronic device.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment, the electronic device is not limited to those described above.

It should be understood that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related components. It is to be understood that a singular form of a noun corresponding to an item may include one or more of the things, unless the relevant context clearly indicates otherwise. As used herein, "A or B," "at least one of A and B," "at least one of A or B," "A, B or C," "at least one of A, B and C," and "at least one of A, B, or C," each of which may include any one of the items listed together in the corresponding one of the phrases, or all possible combinations thereof. Terms such as "1st" and "2nd," or "first" and "second" may be used to simply distinguish a corresponding component from other components, and do not limit the components in other aspects (e.g., importance or order). It is to be understood that if a component (e.g., a first component) is referred to, with or without the term "operatively" or "communicatively," as "coupled with," "coupled to," "connected with," or "connected to" another component (e.g., a second component), the component may be coupled with the other component directly (e.g., by wire), wirelessly, or via a third component.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., a program 140) including one or more instructions that are stored in a storage medium (e.g., an internal memory or an external memory) that is readable by a machine (e.g., an electronic device). For example, a processor (e.g., a processor 120) of the machine (e.g., an electronic device 101) may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semipermanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as a memory of the manufacturer's server, a server of the application store, or a relay server.

According to an embodiment, each component (e.g., a module or a program) of the above-described components may include a single entity or multiple entities, and some of the multiple entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An operation method of a ring electronic device (102; 200), comprising:
monitoring a wearing state of the ring electronic device (102; 200);
when the wearing state changes, checking a remaining battery level of the ring electronic device (102; 200); and
displaying the remaining battery level by adaptively blinking a light emitter (271) included in a photoplethysmography (PPG) sensor (270) of the ring electronic device (102; 200).

2. The operation method of the ring electronic device (102; 200) of claim 1, wherein the displaying of the remaining battery level comprises:
comparing a predetermined threshold with the remaining battery level and adaptively blinking the light emitter (271) included in the PPG sensor (270).

3. The operation method of the ring electronic device (102; 200) of any one of claims 1 and 2, wherein the displaying of the remaining battery level comprises:
when the remaining battery level is greater than or equal to the predetermined threshold, blinking a green light-emitting diode (LED) (271-2) included in the PPG sensor (270); and
when the remaining battery level is less than the predetermined threshold, blinking a red LED (271-3) included in the PPG sensor (270).

4. The operation method of the ring electronic device (102; 200) of any one of claims 1 to 3, wherein a number of blinks of the light emitter (271) is based on the remaining battery level.

5. The operation method of the ring electronic device (102; 200) of any one of claims 1 to 4, wherein
the blinking of the green LED (271-2) comprises, when the remaining battery level is greater than or equal to the predetermined threshold, blinking the green LED (271-2) a number of times corresponding to the remaining battery level, and
the blinking of the red LED (271-3) comprises, when the remaining battery level is less than the predetermined threshold, blinking the red LED (271-3) a number of times equal to the remaining battery level minus 1.

6. The operation method of the ring electronic device (102; 200) of any one of claims 1 to 5, wherein the remaining battery level is collected at a predetermined interval.

7. An operation method of a ring electronic device (102; 200), comprising: monitoring shaking information of the ring electronic device (102; 200); when shaking of the ring electronic device (102; 200) is detected, checking a remaining battery level of the ring electronic device (102; 200); and
displaying the remaining battery level by adaptively blinking a light emitter (271) included in a photoplethysmography (PPG) sensor (270) of the ring electronic device (102; 200).

8. A ring electronic device (102; 200), comprising:
a wireless communication circuit (210) configured to transmit and receive a wireless signal;
a processor (220) operatively connected to the wireless communication circuit (210); and
a memory (230) storing instructions,
wherein the instructions, when executed individually or collectively by the processor (220), cause the ring electronic device (102; 200) to:
monitor a wearing state of a ring electronic device;
when the wearing state changes, check a remaining battery level of the ring electronic device; and
display the remaining battery level by adaptively blinking a light emitter (271) included in a photoplethysmography (PPG) sensor (270) of the ring electronic device.

9. The ring electronic device (102; 200) of claim 8, wherein the instructions, when executed individually or collectively by the processor (220), cause the ring electronic device (102; 200) to:
compare a predetermined threshold with the remaining battery level and adaptively blink the light emitter (271) included in the PPG sensor (270).

10. The ring electronic device (102; 200) of any one of claims 8 and 9, wherein the instructions, when executed individually or collectively by the processor (220), cause the ring electronic device (102; 200) to:
when the remaining battery level is greater than or equal to the predetermined threshold, blink a green light-emitting diode (LED) (271-2) included in the PPG sensor (270); and
when the remaining battery level is less than the predetermined threshold, blink a red LED (271-3) included in the PPG sensor (270).

11. The ring electronic device (102; 200) of any one of claims 8 to 10, wherein a number of blinks of the light emitter (271) is based on the remaining battery level.

12. The ring electronic device (102; 200) of any one of claims 8 to 11, wherein the instructions, when executed individually or collectively by the processor (220), cause the ring electronic device (102; 200) to:
when the remaining battery level is greater than or equal to the predetermined threshold, blink the green LED (271-2) a number of times corresponding to the remaining battery level, and
when the remaining battery level is less than the predetermined threshold, blink the red LED (271-3) a number of times equal to the remaining battery level minus 1.

13. The ring electronic device (102; 200) of any one of claims 8 to 12, wherein the remaining battery level is collected at a predetermined interval.

14. The ring electronic device (102; 200) of any one of claims 8 to 13, wherein the instructions, when executed individually or collectively by the processor (220), cause the ring electronic device (102; 200) to:
monitor a wearing state of the ring electronic device based on shaking information of the ring electronic device (102; 200).
